# EUROPEAN PATENT APPLICATION

(11) **EP 4 756 418 A1**
(43) Date of publication of application: **10.06.2026**
(21) Application number: 24851458.0
(22) Date of filing: 02.07.2024
(51) Int. Cl.: G01N 27/04, A01G 7/00, A01K 29/00

(54) **BIOLOGICAL SENSOR AND BIOLOGICAL MANAGEMENT SYSTEM**

(30) Priority: 04.08.2023 JP 2023127860
(71) Applicant: Sony Semiconductor Solutions Corporation, Atsugi-shi, Kanagawa 243-0014 (JP)
(72) Inventor: YOSHINO, Yoshitaka, Atsugi-shi, Kanagawa 243-0014 (JP)
(74) Representative: D Young & Co LLP
(86) International application number: PCT/JP2024/023873
(87) International publication number: WO 2025/033027

(57) **Abstract**

A biological sensor according to an embodiment of the present technology includes a first antenna conductor, a second antenna conductor, and a power detection unit. The second antenna conductor is a conductor different from the first antenna conductor, and is electrically coupled to an organism connected to an earth ground directly or by capacitive coupling. The power detection unit detects AC power to be generated between the first antenna conductor and the second antenna conductor in accordance with a resistance component of the organism relative to the earth ground.

## Description

### Technical Field

The present technology relates to biological sensors that detect states of organisms, and to biological management systems.

### Background Art

Hitherto, technologies for detecting states of organisms have been developed. For example, Patent Literature 1 describes a growth diagnostic apparatus to be applied to vascular plants. This growth diagnostic apparatus is provided with internal-information measuring sensors that measure data on internal information indicating growth states of the vascular plants. As the internal-information measuring sensors, for example, sensors that measure acoustic emissions to be used for evaluating health of the vascular plants, and sensors that measure data on transpiration flow rates, diameters of stems, hardness of the stems, biopotential, and the like of the vascular plants are used (paragraphs [0002], [0020], [0029], [0135], etc., of the specification of Patent Literature 1).

### Citation List

### Patent Literature

Patent Literature 1: Japanese Patent Application Laid-open No. 2017-51125

### Disclosure of Invention

### Technical Problem

The sensors described in Patent Literature 1 detect states of plants. Also for humans and other animals, technologies for detecting their states have been developed. Such technologies for detecting states of organisms are expected to be applied to various fields.

As an example of methods of detecting a state of an organism, there is a method of directly detecting weak signals to be emitted by the organism itself. However, it may be difficult to achieve sufficient detection accuracy. In addition, in order to detect such signals, for example, a specialized measuring instrument or the like may be needed, which may complicate configurations of apparatuses.

In view of the circumstances as described above, the present technology has been made to achieve an object to provide a biological sensor and a biological management system that are capable of detecting states of organisms easily and with high accuracy.

### Solution to Problem

In order to achieve the above-mentioned object, a biological sensor according to an embodiment of the present technology includes a first antenna conductor, a second antenna conductor, and a power detection unit.

The second antenna conductor is a conductor different from the first antenna conductor, and is electrically coupled to an organism connected to an earth ground directly or by capacitive coupling.

The power detection unit detects AC power to be generated between the first antenna conductor and the second antenna conductor in accordance with a resistance component of the organism relative to the earth ground.

This biological sensor is provided with the first antenna conductor and the second antenna conductor that is connected to the earth ground via the organism. The AC power to be generated between these antenna conductors in accordance with the resistance component of the organism relative to the earth ground is detected. This enables relatively high AC power to be obtained despite a simple configuration, and a state of the organism can be detected easily and with high accuracy.

The biological sensor may further include
a signal generator that applies a predetermined AC signal to the first antenna conductor.

A frequency of the predetermined AC signal may be set in accordance with frequency characteristics of the resistance component of the organism relative to the earth ground.

The organism may be a plant.

In this case, the frequency of the predetermined AC signal may be set to a frequency of 5 Hz or more and 3 MHz or less.

The predetermined AC signal may have any of a rectangular wave, a sine wave, and a triangular wave.

The signal generator may include a ground portion that provides reference potential for the predetermined AC signal.

In this case, the ground portion may be electrically coupled to the organism.

The signal generator may include a ground portion that provides reference potential for the predetermined AC signal.

In this case, the ground portion may be connected to the earth ground.

The signal generator may include a ground portion that provides reference potential for the predetermined AC signal.

In this case, the ground portion may be in a state electrically floating from the earth ground.

The power detection unit may include
a rectifier circuit that rectifies the AC power to be generated between the first antenna conductor and the second antenna conductor, and
a DC detection circuit that detects DC power to be output from the rectifier circuit.

The DC detection circuit may be either one of a voltmeter that detects output voltage of the rectifier circuit, and an ammeter that detects output current of the rectifier circuit.

The DC detection circuit may be a voltmeter that detects output voltage of the rectifier circuit.

In this case, an internal resistor of the voltmeter may be adjusted to achieve predetermined sensor sensitivity.

The organism may be a plant.

In this case, a resistance value of the internal resistor of the voltmeter may be 1 kQ or more and 10 MQ or less.

The organism may be a plant.

In this case, the second antenna conductor may be attached to an aboveground part of the plant.

The second antenna conductor may have either one of a contact structure that presses an end portion of the second antenna conductor against an outside of the plant, and a needle structure that inserts the end portion of the second antenna conductor into an inside of the plant.

The organism may be a human or an animal.

In this case, the second antenna conductor may be attached to the body of the human or the body of the animal.

The first antenna conductor may be set to have a length corresponding to 1/4 of a wavelength of a predetermined AC component among AC components to be induced in the organism.

The biological sensor may use an energy harvesting apparatus as a power supply.

A biological management system according to another embodiment of the present technology includes at least one biological sensor and a management apparatus.

The at least one biological sensor includes
a first antenna conductor,
a second antenna conductor that is a conductor different from the first antenna conductor, and is electrically coupled to an organism connected to an earth ground directly or by capacitive coupling, and
a power detection unit that detects AC power to be generated between the first antenna conductor and the second antenna conductor in accordance with a resistance component of the organism relative to the earth ground.

The management apparatus performs a management operation on the basis of a result of detection by the at least one biological sensor.

### Brief Description of Drawings

[Fig. 1] A schematic diagram showing an example of a configuration of a biological sensor according to a first embodiment of the present technology.
[Fig. 2] A schematic explanatory circuit diagram showing operation of a monopole antenna.
[Fig. 3] A schematic explanatory circuit diagram showing operation of the biological sensor.
[Fig. 4] A block diagram showing the example of the configuration of the biological sensor.
[Fig. 5] A circuit diagram showing an example of a configuration of a power detection unit.
[Fig. 6] An explanatory graph showing characteristics of diodes of a rectifier circuit.
[Fig. 7] An explanatory table showing the characteristics of the diodes of the rectifier circuit.
[Fig. 8] A schematic circuit diagram showing an example of connection between the biological sensor and a plant.
[Fig. 9] A schematic view illustrating the example of the connection between the biological sensor and the plant, the connection being shown in Fig. **8****.**
[Fig. 10] A graph showing an example of results of monitoring by the biological sensor.
[Fig. 11] A schematic diagram showing an example of a configuration of a biological management system using the biological sensor.
[Fig. 12] A schematic circuit diagram showing another example of the connection between the biological sensor and the plant.
[Fig. 13] A schematic circuit diagram showing a still another example of the connection between the biological sensor and the plant.
[Fig. 14] A schematic circuit diagram showing an example of a configuration of a biological sensor according to a second embodiment.
[Fig. 15] A schematic view illustrating examples of applying the biological sensor to a human body.
[Fig. 16] A schematic view illustrating an example of applying the biological sensor to an animal.
[Fig. 17] A schematic circuit diagram showing an example of a configuration of a biological sensor according to another embodiment.

### Mode(s) for Carrying Out the Invention

Hereinbelow, embodiments according to the present technology are described with reference to the drawings.

### [Outline of Biological Sensor]

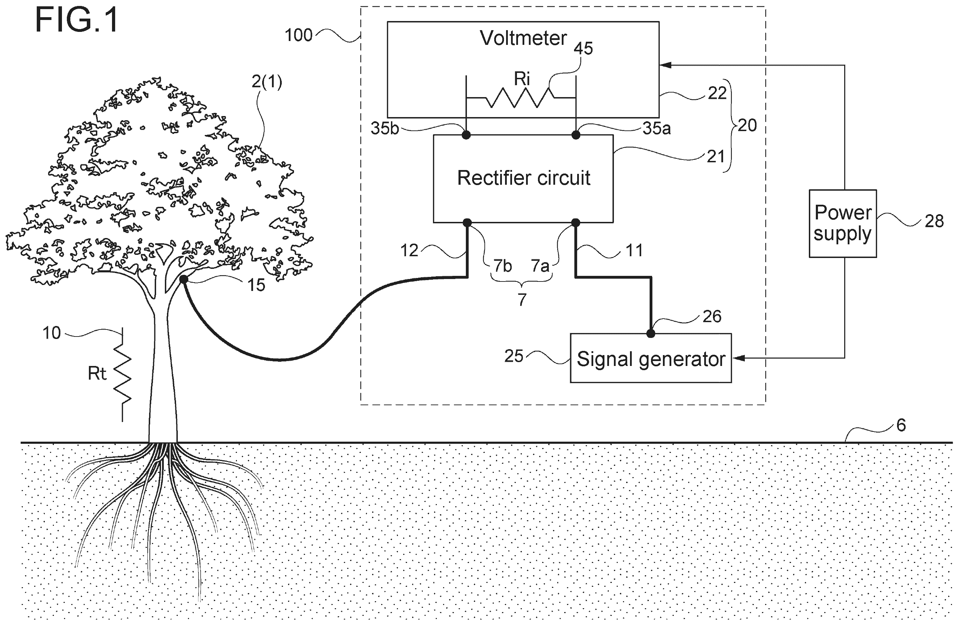

Fig. 1 is a schematic diagram showing an example of a configuration of a biological sensor according to a first embodiment of the present technology. The biological sensor 100 is a sensor that detects a state of an organism 1. The organism 1 contains a resistance component 10 that acts as an electrical resistor. The state of the organism 1, the state being detected by the biological sensor 100, includes internal and external states of the organism 1, the states being estimated from the resistance component 10 of the organism 1 to be detected. The biological sensor 100 detects the state of the organism 1 by detecting a variation in the resistance component 10 as an electrical signal.

In this embodiment, the organism 1 is a plant 2. For example, the resistance component 10 varies in response to variations in water content and concentration of electrolytes (such as sap) (hereinbelow, referred to as electrolyte concentration) contained in the plant 2. From such variations in the resistance component 10, the biological sensor 100 detects states of the water content, the electrolyte concentration, and the like of the plant 2. In Fig. 1, a tree growing in the ground is schematically illustrated as an example of the plant 2. In addition, the resistance component 10 of the plant 2 to be detected by the biological sensor 100 is schematically shown using the symbol for a resistor element. Hereinbelow, a resistance value of the resistance component 10 may be denoted by Rt.

The biological sensor 100 includes a first antenna conductor 11, a second antenna conductor 12, a power detection unit 20, and a signal generator 25. In addition, the biological sensor 100 is connected to a power supply 28, and operates on power to be supplied from the power supply 28. In the biological sensor 100, AC power to be generated between the first antenna conductor 11 and the second antenna conductor 12 is detected by the power detection unit 20.

The first antenna conductor 11 is an antenna that receives a signal which generates the above-mentioned AC power. In this embodiment, the first antenna conductor 11 is connected to the signal generator 25. In this case, the first antenna conductor 11 can be regarded as an antenna to which a signal from the signal generator 25 is input.

The second antenna conductor 12 is electrically coupled to the plant 2 connected to an earth ground directly or by capacitive coupling. It can also be said that the second antenna conductor 12 is connected to the earth ground (hereinbelow, referred to as earth ground 6) via the plant 2. The resistance component 10 of the plant 2 is, for example, a component that serves as the electrical resistor from a connection part between the second antenna conductor 12 and the plant 2 to the earth ground 6.

In the example shown in Fig. 1, the ground where the plant 2 grows functions as the earth ground 6. In this case, the plant 2 is connected directly to the earth ground 6. Note that, the present technology is applicable also to plants other than those growing in the ground. For example, when the plant 2 is grown in a container such as a planter, roots, the trunk, branches, leaves, and the like of the plant 2 are connected to the earth ground 6 by capacitive coupling. In this case, the second antenna conductor 12 is connected to the earth ground 6 via the plant 2 by capacitive coupling.

In addition, the earth ground 6 need not necessarily be the actual ground. For example, when the plant 2 is grown indoors, among parts around the plant 2, parts at which potential does not substantially vary (such as a metal structural member or grounding lines prepared together with electrical wiring) functions as the earth ground 6.

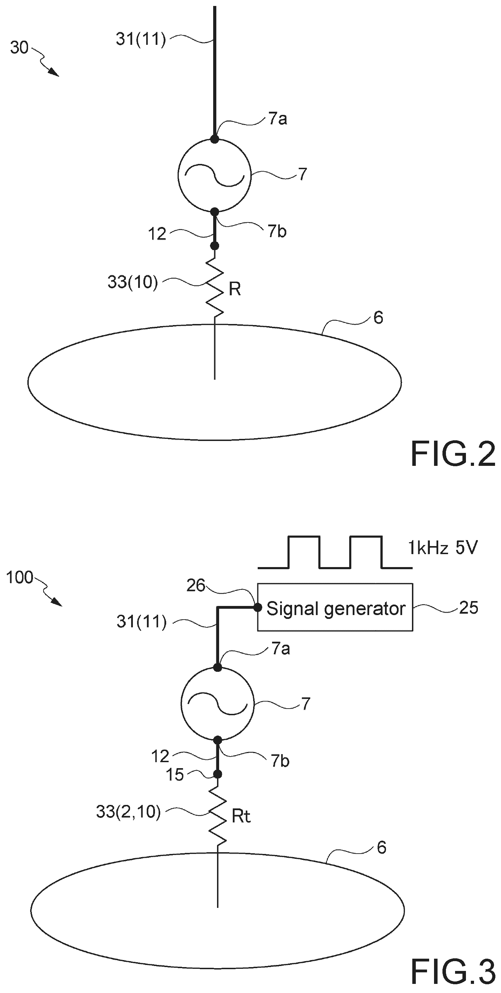

The power detection unit 20 detects the AC power to be generated between the first antenna conductor 11 and the second antenna conductor 12 in accordance with the resistance component 10 of the plant 2 relative to the earth ground 6. As described below with reference, for example, to Fig. 3, an AC electrical signal that varies in accordance with the resistance component 10 of the plant 2 (hereinbelow, referred to as a detection signal) is generated between the first antenna conductor 11 and the second antenna conductor 12. By detecting the AC power of the detection signal, the power detection unit 20 detects the states of the plant 2 (such as those of water content and the electrolyte concentration), the states being indicated by the resistance component 10.

As shown in Fig. 1, the power detection unit 20 rectifies, via its rectifier circuit 21, the detection signal to be generated between the first antenna conductor 11 and the second antenna conductor 12, and measures, via its voltmeter 22, a voltage level of a DC electrical signal to be output from the rectifier circuit 21. In such a way, in the power detection unit 20, the AC power of the detection signal is detected by detecting the voltage level of the rectified detection signal.

The signal generator 25 applies a predetermined AC signal to the first antenna conductor 11. Hereinbelow, this predetermined AC signal is referred to as an applied signal. In this embodiment, in response to the application of the applied signal from the signal generator 25 to the first antenna conductor 11, the AC electrical signal (detection signal) in accordance with the applied signal and the resistance component 10 of the plant 2 is generated between the first antenna conductor 11 and the second antenna conductor 12. It can be said that the biological sensor 100 is a sensor that actively generates the detection signal by using the signal generator 25 in such a way.

### [Operation of Monopole Antenna]

Fig. 2 is a schematic explanatory circuit diagram showing operation of a monopole antenna. Here, for describing the operation of the biological sensor 100, first, operation of a monopole antenna 30 is described. The monopole antenna 30 is an antenna including a feed point 7 between its single receiving antenna 31 and the earth ground 6. Note that, the feed point 7 is a pair of terminal parts of current paths. Specifically, the feed point 7 includes an antenna-side feed point 7a that is connected to the receiving antenna, and a ground-side feed point 7b that is connected to the earth ground 6.

In the monopole antenna 30 shown in Fig. 2, a resistor 33 is connected between the feed point 7 and the earth ground 6. In other words, the feed point 7 is formed between the resistor 33 connected to the earth ground 6 and the receiving antenna 31. In this monopole antenna 30, for example, in response to reception of an electromagnetic wave by the receiving antenna 31, current corresponding to this electromagnetic wave flows through the receiving antenna 31. In addition, image current corresponding to the current that flows through the receiving antenna 31 flows through the resistor 33 connected to the earth ground 6. As a result, the AC electrical signal is generated at the feed point 7 (between antenna-side feed point 7a and ground-side feed point 7b).

Power (the AC power) of the AC electrical signal to be generated at the feed point 7 has a value corresponding to a resistance value of the resistor 33. For example, as a resistance value R of the resistor 33 becomes smaller, the image current to flow through the resistor 33 becomes higher. Thus, the AC power to be generated at the feed point 7 becomes higher. In contrast, as the resistance value R of the resistor 33 becomes higher, the image current to flow through the resistor 33 becomes lower. Thus, the AC power to be generated at the feed point 7 becomes lower. In such a way, in the monopole antenna 30, the AC power to be generated at the feed point 7 functions as a parameter that indicates the resistance value R of the resistor 33.

The inventor of the present technology has discovered that the resistance component 10 of the organism 1 can be detected by utilizing such characteristics of the monopole antenna 30, and has developed the biological sensor 100. When the configuration of the monopole antenna 30 is applied to the biological sensor 100, the receiving antenna 31 corresponds to the first antenna conductor 11, and a conductive part connecting the feed point 7 (ground-side feed point 7b) and the resistor 33 to each other corresponds to the second antenna conductor 12. In addition, the resistor 33 can be regarded as the resistance component 10 of the organism 1 (plant 2) that is connected to the earth ground 6 directly or by capacitive coupling.

Here, a case where the electromagnetic wave is received by the monopole antenna 30 is investigated. In general, in order to maximize energy of the electrical signal to be induced at the feed point 7, it is necessary to set a length of the receiving antenna 31 to correspond to 1/4 of a wavelength λ of the electromagnetic wave to be received.

Note that, when the resistance value (impedance) of the resistor 33 is sufficiently high at a frequency of the electromagnetic wave to be received, an electrical signal of sufficient strength may not be induced at the feed point 7. Thus, in order that the organism 1 with the resistance component 10 functions as part of the monopole antenna 30, it is necessary to adjust the frequency of the electromagnetic wave to be received to a frequency corresponding to the resistance component 10 of the organism 1. This means that the length of the receiving antenna 31 is set in accordance with the resistance component 10 of the organism 1.

When the organism 1 to be measured is the plant 2, it can be said that the resistance value Rt of the resistance component 10 is sufficiently low generally within the AM band (from 1 kHz to 3 MHz). In other words, in order that the plant 2 functions as part of the monopole antenna 30, the frequency of the electromagnetic wave to be received is desired to be 3 MHz or less.

As in the case where the plant 2 serves as an antenna in the AM band, the wavelength of the electromagnetic wave to be received is very long. For example, when the frequency is 3 MHz, 1/4 of the wavelength λ is approximately 25 m. Thus, it is difficult to configure the receiving antenna 31 corresponding to this wavelength. In view of such circumstances, in this embodiment, a configuration in which an AC signal at the frequency that enables the plant 2 to function as part of an antenna is directly applied to the first antenna conductor 11 that corresponds to the receiving antenna 31 is employed.

### [Operation of Biological Sensor]

Fig. 3 is a schematic explanatory circuit diagram showing the operation of the biological sensor. The biological sensor 100 shown in Fig. 3 is a simplified illustration of the biological sensor 100 shown in Fig. 1 in accordance with the illustration of the monopole antenna 30 shown in Fig. 2. Note that, the power detection unit 20 is not shown.

The first antenna conductor 11 connects the feed point 7 (antenna-side feed point 7a) and the signal generator 25 to each other. Further, the signal generator 25 applies, to the first antenna conductor 11, the AC signal (applied signal) at the frequency that enables the plant 2 to function as part of an antenna. With this, the first antenna conductor 11 and the signal generator 25 function as the receiving antenna 31 in a monopole-antenna structure.

The second antenna conductor 12 is electrically coupled to the plant 2, and connects the feed point 7 (ground-side feed point 7b) and the plant 2 to each other. In addition, the plant 2 being the resistor 33 is connected to the earth ground 6 via its roots. With this, the second antenna conductor 12 and the plant 2 function as a part that connects the feed point 7 to the earth ground 6 via the resistor 33 in the monopole-antenna structure.

The applied signal at a frequency suitable for the plant 2 is output from the signal generator 25. In an example shown in Fig. 3, as the applied signal, a rectangular wave with a frequency of 1 kHz and an amplitude of 5 V is output and applied to the first antenna conductor 11. In this case, the earth ground 6 is connected to the feed point 7 of the biological sensor 100 in a manner that includes the resistance value Rt of the plant 2. In addition, the resistance value Rt is the resistance value of the resistance component 10 of the plant 2 with respect to the AC signal at the frequency of the applied signal (specifically, 1 kHz).

In such a way, in the biological sensor 100, the first antenna conductor 11 is connected to the signal generator 25, and the second antenna conductor 12 is electrically coupled to the plant 2. In addition, from the signal generator 25, the applied signal at the frequency suitable for the plant 2 is applied to the first antenna conductor 11. With this, similar to the monopole antenna 30 shown in Fig. 2, a structure in which the AC power corresponding to the resistance component 10 (resistance value Rt) of the plant 2 is generated at the feed point 7 between the first antenna conductor 11 and the second antenna conductor 12 is generated is provided.

The resistance value Rt of the plant 2 (resistance component 10) relative to the earth ground 6 is determined by the water content and the electrolyte concentration of the plant 2. Thus, the resistance value Rt varies in response to the variations in the water content and the electrolyte concentration of the plant 2. For this reason, by observing power such as voltage or current of the detection signal to be induced at the feed point 7 with the applied signal applied, amounts of the variations depending on the water content and the electrolyte concentration of the plant 2 can be observed.

As described with reference to Fig. 1, in this embodiment, the energy of the AC detection signal to be induced at the feed point 7 is rectified by the rectifier circuit 21, and the DC voltage to be output from the rectifier circuit 21 is detected by the voltmeter 22. Monitoring the DC voltage generated in such a way through the rectification of the detection signal enables the states of the water content, the electrolyte concentration, and the like of the plant 2 to be visualized as voltage values.

### [Configuration of Biological Sensor]

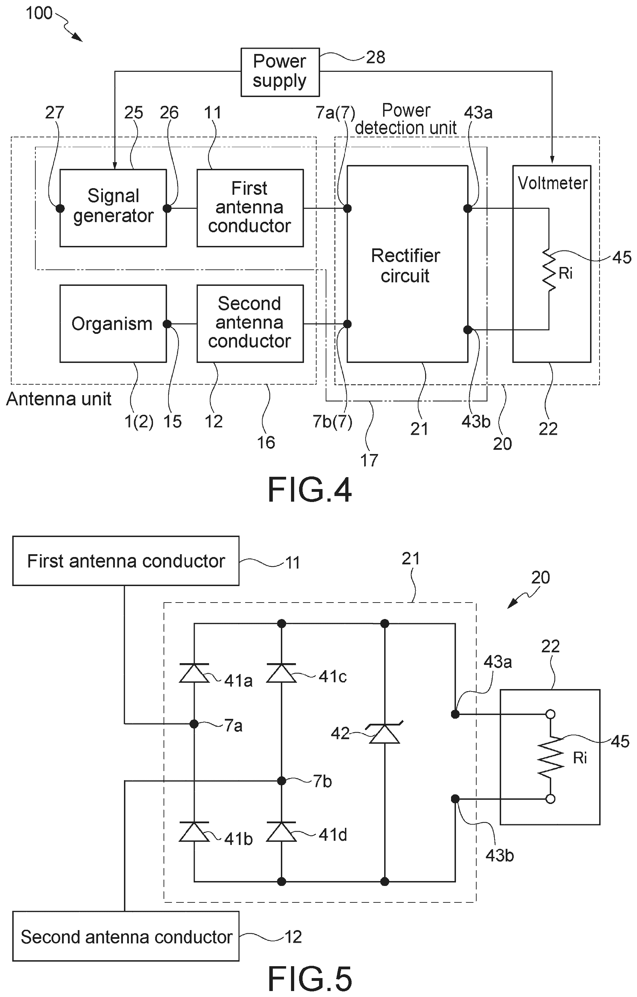

Fig. 4 is a block diagram showing the example of the configuration of the biological sensor 100. Now, respective configurations of the first antenna conductor 11, the second antenna conductor 12, the power detection unit 20, and the signal generator 25 that constitute the biological sensor 100 are described in detail.

### [First Antenna Conductor]

The first antenna conductor 11 is a conductor to which the applied signal is applied from the signal generator 25, and functions as a current path that connects the signal generator 25 and the feed point 7 to each other. One of end portions of the first antenna conductor 11 is connected to an output terminal 26 of the signal generator 25. Meanwhile, another one of the end portions of the first antenna conductor 11 functions as the antenna-side feed point 7a, and is connected to one of input terminals of the rectifier circuit 21.

In this embodiment, the first antenna conductor 11 functions as an input antenna to which the applied signal is directly input from the signal generator 25. In other words, in the biological sensor 100, it is unnecessary to receive, for example, the electromagnetic wave at the frequency suitable for the plant 2. Thus, a length of the first antenna conductor 11 may be arbitrarily set in accordance, for example, with sizes and circuit configurations of the biological sensor 100.

### [Signal Generator]

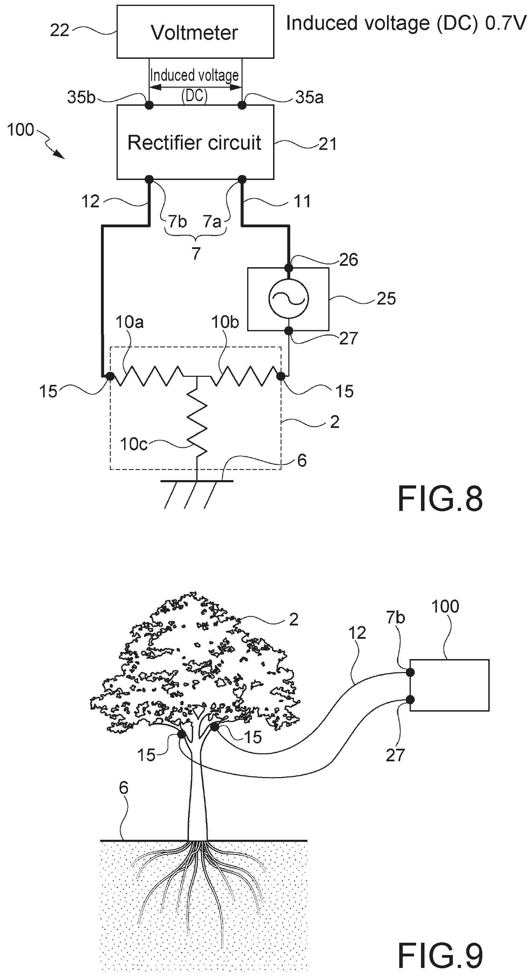

The signal generator 25 is a signal generating circuit that outputs the applied signal being the predetermined AC signal, and includes the output terminal 26 and a ground terminal 27. The output terminal 26 is a terminal that outputs the applied signal, and is connected to the first antenna conductor 11. The ground terminal 27 is a terminal that is connected to the ground of the signal generator 25. Potential of the ground terminal 27 is a reference potential for the applied signal (such as a level at which the voltage is zero). Connection destinations of the ground terminal 27 are described in detail below with reference, for example, to Fig. 8, Fig. 12, and Fig. 13. In this embodiment, the ground terminal 27 corresponds to a ground portion that provides the reference potential for the applied signal.

The frequency of the applied signal is set in accordance with frequency characteristics of the resistance component 10 of the plant 2 relative to the earth ground 6. As described above, in order that the resistance value Rt (impedance) of the resistance component 10 contained in the plant 2 is sufficiently low, and that the plant 2 functions as an antenna, the frequency is desired to be within the AM band or lower. Thus, in this embodiment, the frequency of the applied signal is set to a frequency of 5 Hz or more and 3 MHz or less. This enables the image current at the same frequency as that of the applied signal to flow through the plant 2, and the detection signal of sufficient strength is induced at the feed point 7. This enables reliable detection of the variation in the resistance value Rt.

Further, a waveform of the applied signal is, for example, rectangular. In this case, a logic source such as a pulse generator may be utilized as the signal generator 25, which simplifies a circuit configuration of the signal generator 25. Note that, the waveform of the applied signal is not limited, and a sine wave, a triangular wave, or the like may be used. In this case, for example, a function generator that outputs signals with arbitrary waveforms is used as the signal generator 25. This enables, for example, selection of appropriate waveforms in accordance with characteristics and types of the plant 2.

Still further, a center voltage of the amplitude of the applied signal is not limited. The center voltage may be set, for example, to a zero level at which the voltage values are zero, or may be set to voltage other than the zero level by applying a DC offset. In addition, the waveform of the applied signal is not limited, and applied signals with the above-mentioned arbitrary waveforms may be used.

### [Second Antenna Conductor]

The second antenna conductor 12 is a conductor that is electrically coupled to the plant 2, and functions as the current path that connects the plant 2 and the feed point 7 to each other. The second antenna conductor 12 is constituted, for example, by using a metal cable or the like. One of end portions of the second antenna conductor 12 serves as an attachment portion 15 that is attached to the plant 2. Meanwhile, another one of the end portions of the second antenna conductor 12 functions as the ground-side feed point 7b, and is connected to another one of the input terminals of the rectifier circuit 21.

The second antenna conductor 12 is attached to an aboveground part of the plant 2. The aboveground part of the plant 2 refers to a part above a ground surface. For example, the trunk, the branches, the leaves, and the like of the plant 2 correspond to the aboveground parts. For example, in Fig. 1, the attachment portion 15 of the second antenna conductor 12 is attached to the branch of the plant 2. Note that, the roots of the plant 2 are underground parts of the plant 2. Attaching the second antenna conductor 12 to the aboveground part eliminates a need to make a direct connection between the second antenna conductor 12 and the earth ground 6. This enables proper measurement of the resistance value Rt of the resistance component 10 of the plant 2.

The attachment portion 15 is a probe electrode for holding an end portion of the cable constituting the second antenna conductor 12 in contact with the plant 2. Typically, metal is used for a part of the attachment portion 15, the part being held in contact with the plant 2. The attachment portion 15 includes, for example, either one of a contact structure that presses the end portion of the second antenna conductor 12 against an outside of the plant 2, and a needle structure that inserts the end portion of the second antenna conductor 12 into an inside of the plant 2.

The attachment portion 15 with the contact structure is, for example, a probe electrode of a type that presses a metal electrode (the end portion of the second antenna conductor 12) against an attachment object by utilizing a spring, a fastener, or the like. For example, a clip structure that utilizes a spring (for example, a metal clip such as an alligator clip) is used as the attachment portion 15 with the contact structure. Alternatively, the attachment portion 15 may be configured, for example, to have a structure that presses the metal electrode against the attachment object by twisting a wire wrapped around the attachment object, or a structure that presses the metal electrode against the attachment object by using a resin cable tie. Still alternatively, the contact structure to be used as that of the attachment portion 15 may be any structure capable of holding the metal electrode in contact with the attachment object. Use of the attachment portion 15 with the contact structure enables the second antenna conductor 12 to be easily attached to the plant 2. For example, in a case where the electrode is not directly exposed to moisture such as rain as in indoor cultivation in a greenhouse, the attachment portion 15 with the contact structure can be used without any problem.

The attachment portion 15 with the needle structure is, for example, a probe electrode of a type that inserts a needle-shaped electrode into an inside of the trunk or the branch of the plant 2. In this case, direct contact with the inside of the plant 2 can be made. For example, in the attachment portion 15 with the clip structure, entry of moisture such as rain between electrodes that clamp the plant 2 causes variation in contact resistance. In contrast, since the attachment portion 15 with the needle structure is used by inserting the needle-shaped electrode or the like into the plant 2, the second antenna conductor 12 can be held in direct contact with the plant 2. Thus, influence of the moisture such as rain can be reduced. This enables stable contact even outdoors, for example.

As a material of the metal constituting the attachment portion 15, in order to reduce susceptibility, for example, to corrosion from the moisture, a gold-plated metal material, stainless steel, or the like is desired to be used. In addition to the structures of the attachment portion 15, any other structures capable of electrically coupling a body (cable part) of the second antenna conductor 12 and the plant 2 to each other may be used.

By attaching the second antenna conductor 12 of the biological sensor 100 to the plant 2, an antenna unit 16 including two antenna elements is formed. Specifically, the signal generator 25 and the first antenna conductor 11 connected to the signal generator 25 function as an antenna element that is connected to the antenna-side feed point 7a and receives the AC signal. In addition, the plant 2 and the second antenna conductor 12 connected to the plant 2 function as another antenna element that connects the ground-side feed point 7b to the earth ground 6. The antenna unit 16 including these two antenna elements enables the AC power corresponding to the resistance component 10 of the plant 2 to be induced at the feed point 7.

Fig. 5 is a circuit diagram showing an example of the configuration of the power detection unit 20. The power detection unit 20 is a circuit that rectifies the AC power to be generated at the feed point 7, and detects a level of the power. The power detection unit 20 includes the rectifier circuit 21 and the voltmeter 22.

### [Rectifier Circuit]

The rectifier circuit 21 rectifies the AC power to be generated between the first antenna conductor 11 and the second antenna conductor 12 (feed point 7). In other words, the rectifier circuit 21 converts the power of the AC detection signal to be generated at the feed point 7 to DC power. Thus, output from the rectifier circuit 21 is a DC signal that indicates the power level of the detection signal.

In the biological sensor 100, in detection of a variation in the power level of the detection signal, accuracy of the detection can be enhanced as the voltage to be output from the rectifier circuit 21 becomes higher. For example, a full-wave rectifier circuit is capable of increasing the voltage of the AC signal and capturing an entirety of a waveform, and hence is efficient even when including losses in diodes to be used for the rectification. In addition, it is important that leakage current of the diodes under reverse bias be very small in the rectifier circuit 21. The full-wave rectifier circuit is suitable when there is leakage current. For these reasons, the full-wave rectifier circuit is desired to be used as the rectifier circuit 21. Note that, the configuration of the rectifier circuit 21 is not limited thereto, and a half-wave rectifier circuit, a voltage-multiplier circuit, a Cockcroft-Walton circuit, or the like may also be used.

The rectifier circuit 21 shown in Fig. 5 is configured as the full-wave rectifier circuit. The rectifier circuit 21 includes four diodes 41a, 41b, 41c, and 41d, a Zener diode 42, and output terminals 43a and 43b. The diodes 41a and 41b are connected in series, and the diodes 41c and 41d are connected in series. A junction between an anode of the diode 41a and a cathode of the diode 41b corresponds to the antenna-side feed point 7a to which the first antenna conductor 11 is connected. In addition, a junction between an anode of the diode 41c and a cathode of the diode 41d corresponds to the ground-side feed point 7b to which the second antenna conductor 12 is connected.

A junction between a cathode of the diode 41a and a cathode of the diode 41c is connected to the one output terminal 43a, and a junction between an anode of the diode 41b and an anode of the diode 41d is connected to the other output terminal 43b. Although the rectifier diodes 41a, 41b, 41c, and 41d are constituted by discrete diodes, these diodes may be constituted by dedicated ICs.

In addition, the Zener diode 42 is connected in parallel between the output terminals 43a and 43b. The Zener diode 42 functions as an electrostatic protection component that discharges static electricity in a case where high voltage such as static electricity occurs. Instead of the Zener diode 42, a varistor for electrostatic protection may be provided. Note that, the electrostatic protection component such as the Zener diode 42 need not necessarily be provided depending on configurations of the biological sensor 100.

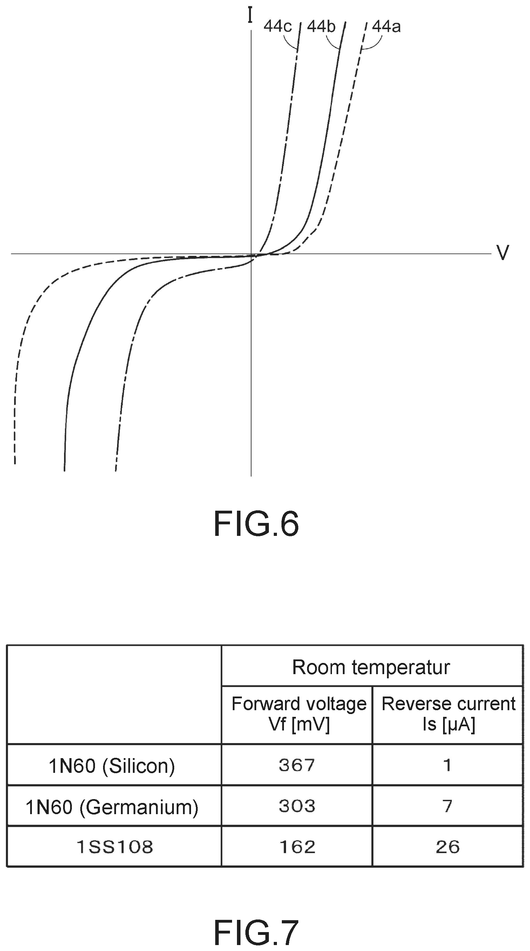

Fig. 6 is an explanatory graph showing characteristics of the diodes of the rectifier circuit 21. Fig. 7 is an explanatory table showing the characteristics of the diodes of the rectifier circuit 21. Fig. 6 and Fig. 7 show results of measurements of forward voltage Vf and reverse current Is of the rectifier diodes used in the rectifier circuit 21. The measurements were taken for both silicon and germanium diodes of model 1N60, and evaluations were performed on germanium diodes of another model 1SS108 from different manufacturers. In Fig. 6, a curve 44a represents characteristics of 1N60 (silicon), a curve 44b represents characteristics of 1N60 (germanium), and a curve 44c represents characteristics of 1SS108 (germanium).

Current that flows in response to application of voltage in a reverse direction of the diodes is the reverse current Is. Measurement data shown in Fig. 7 is data when 10 V is applied in the reverse direction of the diodes. The forward voltage Vf is voltage when forward current (1 mA) starts to flow through the diodes.

It was found that, when output of the above-described antenna unit 16 was rectified, the 1N60 (silicon) diode that blocks flow of reverse current was capable of extracting more power than diodes with low forward turn-on voltage. In addition, the reverse current Is that was generated in response to the application of the forward voltage Vf of the diodes in the reverse direction was evaluated. Given that the signal to be rectified is AC, and that the data shown in Fig. 7 is for 10 V, the reverse current Is in response to the application of the same voltage as Vf is calculated to be 0.036 µA for 1N60 (silicon), 0.21 µA for 1N60 (germanium), and 0.5 µA for 1SS108 (germanium).

Thus, ratios of the reverse current Is at the forward voltage Vf to the forward current (1 mA) are 1/27778 for 1N60 (silicon), 1/4762 for 1N60 (germanium), and 1/2000 for 11SS108 (germanium). In other words, it is necessary for the ratios of the diodes that are used in the rectifier circuit 21 to be more than approximately 4700 times. The ratios are desired to be 10000 or more. As a result, 1N60 (silicon) has the most suitable characteristics among the exemplified three diodes.

In addition, considering the characteristics of the diodes, the reverse current Is in response to the application of the voltage in the reverse direction should be low. By using the data at 10 V, reverse resistance values are calculated to be 100 MΩ for 1N60 (silicon), 1.43 MΩ for 1N60 (germanium), 0.38 MΩ for 1SS108 (germanium). In other words, the resistance values that block the flow of the current in the reverse direction are desired to be high. It is necessary for the resistance values of the diodes that are used in the rectifier circuit 21 to be more than 1.43 MΩ. The resistance values are desired to be 10 MΩ or more. As a result, 1N60 (silicon) has the most suitable characteristics among the exemplified three diodes.

### [Voltmeter]

Referring back to Fig. 5, the voltmeter 22 detects the output voltage of the rectifier circuit 21. An internal resistor 45 is provided in the voltmeter 22. The internal resistor 45 is connected between the output terminal 43a and the output terminal 43b of the rectifier circuit 21. Hereinbelow, a resistance value of the internal resistor 45 is denoted by Ri. The resistance value Ri of the internal resistor 45 corresponds to input impedance of the voltmeter 22, which is set to a relatively high value (such as 1 MQ or more).

In the voltmeter 22, voltage between the output terminal 43a and the output terminal 43b is detected as DC voltage to be applied to the internal resistor 45. This enables the DC power that is output from the rectifier circuit 21 to be detected as voltage. In this embodiment, the voltmeter 22 corresponds to a DC detection circuit that detects the DC power to be output from the rectifier circuit 21.

In addition, the resistance value Ri of the internal resistor 45 is a parameter that influences sensitivity of the voltmeter 22. In other words, the sensitivity of the voltmeter 22 depends on the resistance value Ri. For example, as the resistance value Ri becomes higher, a level of the voltage to be detected by the voltmeter 22 becomes higher, which increases sensitivity to a variation in the output of the rectifier circuit 21. Thus, the internal resistor 45 of the voltmeter 22 is adjusted to achieve predetermined sensor sensitivity.

The resistance value Ri of the internal resistor 45 of the voltmeter 22 is desired to be, for example, 1 kQ or more and 10 MQ or less. By setting the resistance value Ri to 1 kΩ or more, a voltage level for detecting the variation in the resistance component 10 of the plant 2 can be maintained, and the water content and the like of the plant 2 can be properly detected. In addition, by setting the resistance value Ri to 10 MQ or less, the sensor sensitivity of the voltmeter 22 can be prevented from unnecessarily increasing, and significant changes in the output from the rectifier circuit 21 can be detected.

For example, in order to closely observe a variation in the state of the plant 2, the resistance value Ri of the internal resistor 45 is set to approximately 10 MΩ. In this case, not only the variations in the water content and the electrolyte concentration of the plant 2, but also, for example, states of contact of the plant 2, for example, with other living beings can be detected. This enables, for example, detection of situations where wild animals such as boars or monkeys approach and, for example, damage the plant 2.

Note that, when the resistance value Ri is set to 10 MQ, readings of the voltmeter 22 may sensitively respond, which may make it difficult to properly detect the variations in the water content and the like. Thus, in order to observe the water content, the electrolyte concentration, and the like of the plant 2, the resistance value Ri of the internal resistor 45 is desired to be set to approximately 2 MQ. Such adjustment of the resistance value Ri enables sensitivity adjustment in accordance, for example, with the characteristics of the plant 2 and application of biological sensor 100. The resistance value Ri of the internal resistor 45 may be set to other values. For example, even outside the above-described range of the resistance value, the resistance value Ri may be set as appropriate in accordance, for example, with the state of the organism 1 to be measured (specifically, plant 2), and the application and use environments of the biological sensor 100.

### [Power Supply]

As shown in Fig. 4, the signal generator 25 and the voltmeter 22 of the biological sensor 100 are driven by the power to be supplied from the power supply 28. For example, a battery is used as the power supply 28. In this case, there is no restriction on where to install the biological sensor 100, and a range of application of the biological sensor 100 can be extended. Alternatively, a commercial power supply may be used as the power supply 28. In this case, concerns such as battery depletion are eliminated, and the state of the plant 2 can be stably monitored.

The biological sensor 100 may use an energy harvesting apparatus as its power supply. As the energy harvesting apparatus, it is possible to use, for example, solar panels and an energy harvester that generates power from vibrations or the like. The power generated by these energy harvesting apparatuses is charged into a storage battery, and then the power is supplied from the storage battery to the biological sensor 100 as appropriate. Alternatively, for example, the AC power to be generated at the feed point 7 of the biological sensor 100 may be charged into the storage battery. Using the energy harvesting apparatus as the power supply in such a way enables the biological sensor 100 to be used over a long period of time in various environments. In addition, a configuration of the power supply 28 is not limited.

Further, as shown in Fig. 4, in the biological sensor 100, the signal generator 25, the first antenna conductor 11, and the rectifier circuit 21 may be configured as a sensor module 17. In this case, the sensor module 17 is provided with an input terminal for connecting the second antenna conductor 12 (terminal that is connected to the ground-side feed point 7b), and a pair of output terminals for connecting the signal generator 25 (output terminal 43a and output terminal 43b of the rectifier circuit 21). In addition, the sensor module 17 is provided with a power terminal for connecting the power supply 28. Using the sensor module 17 enables the biological sensor 100 to be easily configured in accordance with the organism 1 to be an object (for example, plant 2, a human body, or an animal).

### [Connection between Biological Sensor and Plant]

Fig. 8 is a schematic circuit diagram showing an example of connection between the biological sensor 100 and the plant 2. Fig. 9 is a schematic view illustrating the example of the connection between the biological sensor 100 and the plant 2, the connection being shown in Fig. 8. In Fig. 8 and Fig. 9, the ground terminal 27 of the signal generator 25 is electrically coupled to the plant 2. Note that, a part surrounded by dashed lines in Fig. 8 corresponds to the plant 2, and the resistance component 10 (resistance components 10a, 10b, and 10c) shown inside the dashed lines can be regarded as a variable resistor that varies in accordance with the states of the water content and the electrolyte concentration of the plant 2.

The ground terminal 27 of the signal generator 25 is electrically coupled to a part other than the part to which the second antenna conductor 12 is connected among parts of the plant 2. The ground terminal 27 is connected to the plant 2 via, for example, a cable provided with the attachment portion 15 similar to that of the second antenna conductor 12. For example, in Fig. 9, the ground terminal 27 of the signal generator 25 is connected to a branch on a left side of the plant 2 in the illustration, and the second antenna conductor 12 is connected to a branch on a right side of the same in the illustration.

In such a way, both the second antenna conductor 12 and the ground terminal 27 of the signal generator 25 are connected to the plant 2. This connection state is represented by the three resistance components 10a, 10b, and 10c that are constituted by the plant 2. The resistance component 10a is a resistance component that is connected to the second antenna conductor 12. The resistance component 10b is a resistance component that is connected to the ground terminal 27 of the signal generator 25. In addition, the resistance component 10a and the resistance component 10b are connected to the earth ground 6 via the resistance component 10c. Thus, it can be said that the resistance component 10c is a resistance component common to both the second antenna conductor 12 and the ground terminal 27 of the signal generator 25.

Connecting the ground terminal 27 of the signal generator 25 to the plant 2 enables the signal generator 25 to output the applied signal relative to a potential common to the plant 2. In addition, a part that serves as the common potential is formed in the plant 2 (junction between the resistance components 10a and 10b). With this, components of the applied signal are easily reflected in the detection signal to be excited at the feed point 7. Thus, the power level of the detection signal increases. This can also be said that the power level of the detection signal is increased because the plant 2 itself functions as a large ground.

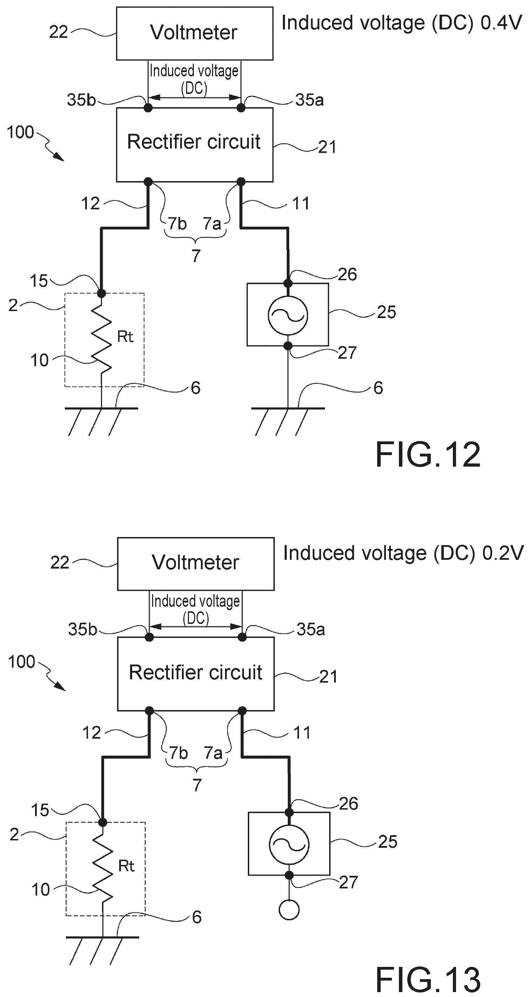

In such a way, in the connection configuration shown in Fig. 8 and Fig. 9, the power to be excited at the feed point 7 increases, which clarifies the variations in the plant 2. This enables the variation in the resistance component 10 (resistance components 10a, 10b, and 10c) to be detected with high accuracy. In addition, an electrode that is connected to the ground terminal 27 of the signal generator 25 is not buried in the ground, and hence there is no risk of soil contamination. Note that, if the electrode is buried in the ground so that the ground terminal 27 is connected to the earth ground 6 (refer to Fig. 12), it is necessary that influence of a potential difference between the ground on which the plant 2 grows and ground in which the electrode is buried be taken into account. In contrast, if the ground terminal 27 is connected to the plant 2, the influence of such potential difference need not necessarily be taken into account.

Actually, both the second antenna conductor 12 and the ground terminal 27 of the signal generator 25 were connected to blue daze, a potted ornamental plant, and the output of the voltmeter 22 was measured. The output of the voltmeter 22 is a value of the DC induced voltage to be output from the rectifier circuit 21. In this measurement, the rectangular wave at the frequency of 1 kHz and the amplitude of 5 V was output as the applied signal, and the resistance value Ri of the internal resistor 45 of the voltmeter 22 was set to 2 MΩ. As a result, the output of the voltmeter 22 was approximately 0.7 V. This is at a relatively higher level than those in other connection examples that are described below with reference to Fig. 12 and Fig. 13. This enables the variation in the resistance component 10 (resistance components 10a, 10b, and 10c) of the plant 2 to be detected with sufficient accuracy.

### [Results of Monitoring]

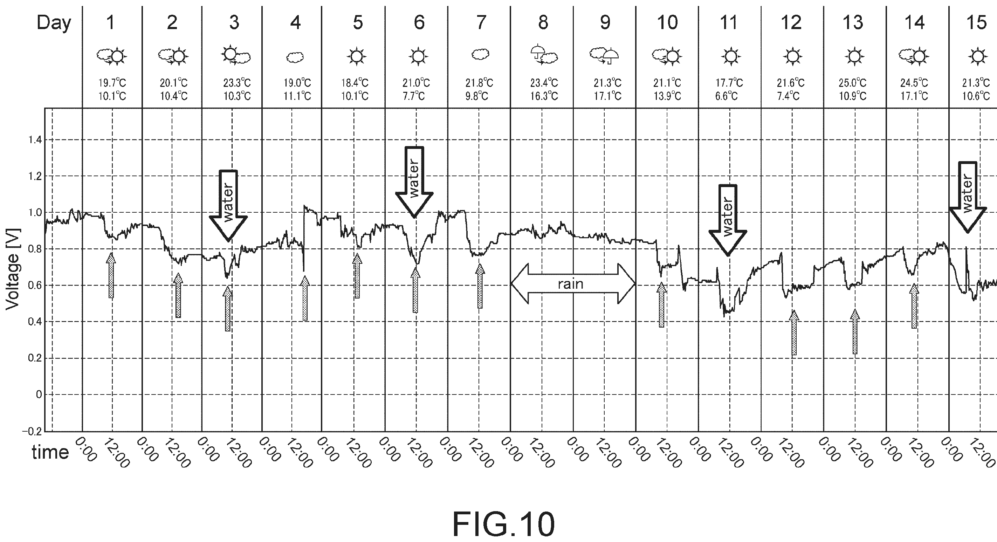

Fig. 10 is a graph showing an example of results of the monitoring by the biological sensor 100. The graph shown in Fig. 10 was generated by monitoring the readings of the voltmeter 22 via the biological sensor 100 connected to the potted blue daze by the connection method shown in Fig. 8 and Fig. 9. In this measurement, the rectangular wave at the frequency of 1 kHz and the amplitude of 5 V was output as the applied signal, and the resistance value Ri of the internal resistor 45 of the voltmeter 22 was set to 2 MΩ.

The abscissa axis represents time. Solid lines represent 0:00 when a date changes, and dashed lines represent 12:00 noon each day. Further, weather, a maximum temperature, and a minimum temperature are also shown for each date. The ordinate axis represents the readings (Voltage [V]) of the voltmeter 22. Still further, thick arrows shown on the graph indicate timings of watering. Hatched arrows indicate timings when photosynthesis is presumed to occur during the day. Yet further, it rained on the 8th day and the 9th day, and hence arrows indicating this period are also shown.

Over the course of a day, as a whole, there was a tendency that the voltage decreased during the day, and the voltage increased at night. For example, it is presumed that, during the day, stomata of the leaves open to cause transpiration to reduce the water content. In this case, the resistance value Rt of the resistance component 10 increases, and the voltage level to be detected by the voltmeter 22 (power level of the detection signal) decreases. In contrast, it is presumed that, at night, the stomata close to suppress the transpiration to cause the moisture to be stored in the plant 2. In this case, the resistance value Rt decreases, and the voltage level increases. The graph is presumed to reflect such detected activity of the plant 2.

In addition, for example, on rainy days, the variation in the voltage is small. This is presumably because humidity remains high all day long, and hence the stomata are closed, and the variations in the water content and the like are small. Further, it was found that, even when watering was performed, the voltage did not immediately increase, and there was a time lag. Using the biological sensor 100 in such a way enables checking of various states of the plant 2, the states involving the variation in the resistance component 10. This enables, for example, determination of optimum timings for fertilizing or watering. Note that, although the experimental results shown in Fig. 10 were obtained by the measurement of a potted plant, the present technology is applicable also to cases of open-field cultivation and the like.

In addition, the biological sensor 100 does not directly apply voltage to the plant 2, and the image current corresponding to the frequency of the applied signal applied to one side of the feed point 7 is induced on a side where the plant 2 is present. While this method does not cause serious damage to the plant 2, the image current to flow through the plant 2 causes moderate stress to the plant 2. As a result, for example, an advantage of an increase in sweetness of fruits such as tomatoes can be expected. In addition, since the voltage corresponding to the image current to flow through the plant 2 is induced, an advantage of repelling pests can be expected.

Note that, it was found that, when the biological sensor 100 was used, the level of the output voltage varied depending also on soil components or types of the plant 2. Thus, for example, before a start of the monitoring, a reference value for the voltage level is desired to be set in accordance with a measurement system. The reference value for the voltage level is a voltage level for determining levels of the water content and the electrolyte concentration of the plant 2 in the measurement system.

For example, an average value of voltage levels that are measured in a properly watered and fertilized state is set as the reference value. In addition, an amount of variation in the voltage levels measured in the same state may be set as a threshold. With this, for example, a determination that watering is needed can be made if the voltage level falls below the threshold relative to the reference value. In addition, a determination that overwatering has been performed can be made if the voltage level falls below the threshold relative to the reference value. In such a way, the voltage level of the biological sensor 100 may be calibrated.

Basically, the biological sensor 100 detects the state of the plant 2 to which the biological sensor 100 is attached. For example, with regard, for example, to a plant group in which roots are in contact with each other underground, it is presumed that states of plants in the group are collectively detected.

For example, if the plant 2 includes a plant that is grown in a planter, such as tomatoes that are planted relatively close together, or if the plant 2 includes plants that are grown side by side, such as grapevines, roots of the plants 2 are presumed to overlap with each other. In these cases, instead of installing the biological sensors 100 on each of the plants 2, the biological sensors 100 may be arranged, for example, respectively in planters, or at ends and a middle of a hedge. In such a way, even without installing the biological sensor 100 on each of the plants 2, if the roots of the plants 2 are in contact with each other, it is also possible to collectively detect states of the plurality of plants 2, and to manage an entirety of the group of the plants.

### [Biological Management System]

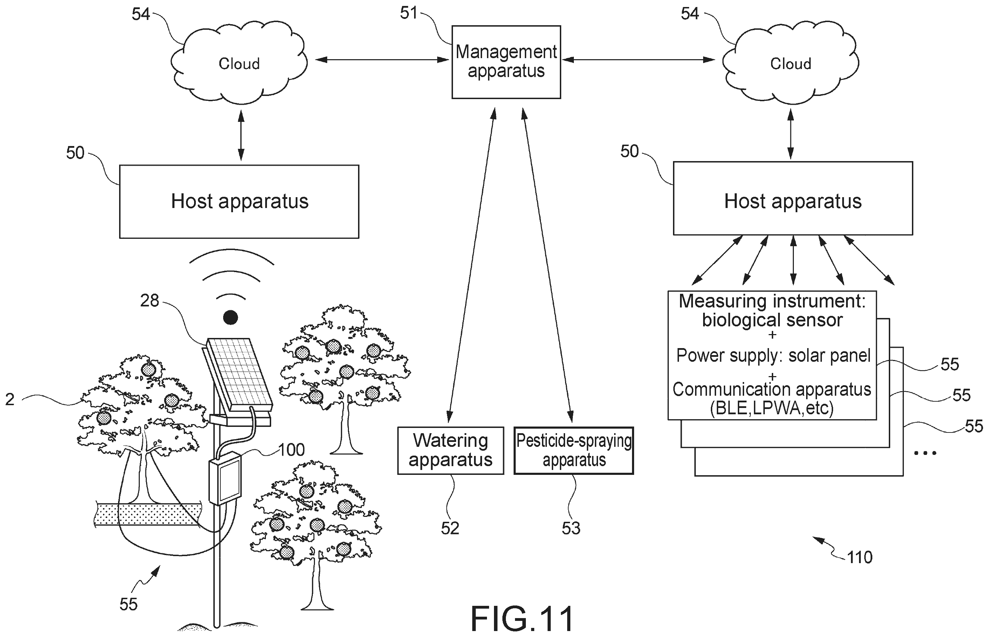

Fig. 11 is a schematic diagram showing an example of a configuration of a biological management system using the biological sensor. This biological management system 110 is a system for managing the plant 2 being the organism 1 with use of the at least one biological sensor 100.

Here, as an example of the biological management system 110, a system in which the biological sensor 100 is arranged in an orchard growing citrus trees and the like. In this case, the plant 2 to be detected by the biological sensor 100 is a fruit tree such as the citrus tree. The biological management system 110 includes the at least one biological sensor 100, a host apparatus 50, a management apparatus 51, a watering apparatus 52, and a pesticide-spraying apparatus 53.

In addition, in the biological management system 110, the biological sensor 100, a communication apparatus (not shown) for communicating with the host apparatus 50, and the power supply 28 for the biological sensor 100 and the communication apparatus are configured as a single sensor unit 55. The second antenna conductor 12 of the biological sensor 100, and the ground terminal 27 of the signal generator 25 are connected to the plant 2 in the orchard via respective cables. Note that, the orchard is outdoors, and hence the second antenna conductor 12 and the ground terminal 27 (neither of which is shown) are connected to the plant 2 via, for example, the probe electrode with the needle structure that is not affected, for example, by rain.

For example, a solar panel is used as the power supply 28. Here, power from the solar panel is charged into an energy storage apparatus such as a secondary battery or a capacitor (not shown), and is supplied as appropriate to the biological sensor 100 and the communication apparatus. In addition, as the communication apparatus, a communication module capable of communication using a short-range communication method such as BLE (Bluetooth Low Energy) or a long-range communication method such as LPWA (Low Power Wide Area-network) is used.

Detected data detected by the biological sensor 100 is transmitted to the host apparatus 50 via the communication apparatus. How often the detected data is transmitted is set in accordance, for example, with an amount of the electric power to be obtained from the solar panel, the characteristics of the plant 2, and application of the biological management system 110. For example, the detected data may be transmitted at an interval of, for example, 5 minutes, 1 hour, or 1 day. The host apparatus 50 receives the detected data transmitted from each of the sensor units 55 (biological sensors 100), and uploads the detected data to a cloud network (cloud 54). The detected data is analyzed in the cloud 54, or transmitted as it is to the management apparatus 51 and then analyzed by the management apparatus 51.

The management apparatus 51 is an apparatus that performs a management operation on the basis of results of the detection by the at least one biological sensor 100. Note that, the management operation refers to an operation for managing the plant 2 or the like to which the biological sensor 100 is connected. Examples of the management operation include controlling a dedicated apparatus for managing the plant 2, and notifying an administrator of the state of the plant 2. Here, the management operation is performed on the basis of results of the analyses of the data detected by the biological sensor 100.

For example, if it is determined that the water content of the plant 2 is insufficient, a notification that the water content is insufficient is transmitted to a terminal apparatus that the administrator uses (such as a PC, a smartphone, or a tablet). In this way, the administrator is alerted. In this case, the administrator may manually operate the watering apparatus 52. Alternatively, the management apparatus 51 may automatically operate the watering apparatus 52. In the automatic mode, for example, the host apparatus 50 communicates to the watering apparatus 52 via the cloud 54 to cause a watering valve to be automatically opened. In this way, water is supplied to the plant. In addition, the management operation can be similarly performed also in a case where the leaves and the like of the plant 2 are damaged by pests and the like. For example, if it is determined that the damage by the pests has occurred, the pesticide-spraying apparatus 53 is activated to splay pesticide as in the case of watering.

As described above, in the biological management system 110, use of the biological sensors 100 enables direct detection of the respective states of the plants 2. Hitherto, humans have judged states of plants on the basis of moisture, air temperature, humidity, weather, and the like. Now, it is possible to automatically provide data to support these judgments, and make determinations of the states of the plants 2. In addition, the management operation can be performed in accordance with the states of the plants at appropriate timings and, in some cases, automatically. This enables the plants 2 to be properly managed to maintain their favorable growth conditions.

### [Other Examples of Connection]

Fig. 12 is a schematic circuit diagram showing another example of the connection between the biological sensor 100 and the plant 2. In the biological sensor 100 shown in Fig. 12, the ground terminal 27 of the signal generator 25 is connected to the earth ground 6. In this state, the ground terminal 27 of the signal generator 25 is connected to a ground common to the plant 2. Note that, the second antenna conductor 12 is electrically coupled to the plant 2. Electrodes to be connected to the earth ground 6 may be, for example, exposed to rain. As a precaution, stainless steel, gold-plated metal, and the like that have low susceptibility to moisture-induced corrosion are desired to be used for electrode parts.

In the example shown in Fig. 12, the signal generator 25 is allowed to output the applied signal relative to potential of the earth ground 6 that serves as the ground common to the plant 2. This increases the power level of the detection signal to be excited at the feed point 7. In addition, by using the earth ground 6 as the reference, the output of the voltmeter 22 is stabilized. When the ground terminal 27 is connected to the earth ground, the output of the voltmeter 22 is higher than, for example, that in a floating state shown in Fig. 13. The output of the voltmeter 22, the output being obtained by a measurement method similar to that in the connection configuration shown in Fig. 8, was approximately 0.4 V.

Fig. 13 is a schematic circuit diagram showing a still another example of the connection between the biological sensor 100 and the plant 2. In the biological sensor 100 shown in Fig. 13, the ground terminal 27 of the signal generator 25 is used in a state electrically floating from the earth ground 6. In this case, the ground terminal 27 is not connected anywhere. Note that, the second antenna conductor 12 is electrically connected to the plant 2.

Under the state in which the ground terminal 27 is electrically floating, a difference between potential of the ground-side feed point 7b and potential of the antenna-side feed point 7a relative to the earth ground 6 appears small. As a result, components of the applied signal are unlikely to be reflected in the detection signal to be excited at the feed point 7. Thus, the power of the detection signal to be induced at the feed point 7 is relatively small. For example, in order to detect a minute variation in the resistance component 10 of the plant 2, the sensitivity of the voltmeter 22 is desired to be increased in advance by setting the internal resistor 45 of the voltmeter 22 to have a high resistance value such as 10 MΩ.

When the ground terminal 27 is floated, the output of the voltmeter 22, the output being obtained by the measurement method similar to that in the connection configuration shown in Fig. 8, was approximately 0.2 V. In such a way, the output of the voltmeter 22, the output being lower than those in other connection configurations, is sufficiently higher than those in configurations that directly detect biopotential. Thus, the variation in the output can be easily detected. In addition, this methods eliminates a need for cables for routing the ground terminal 27, and hence an apparatus configuration can be simplified.

As described hereinabove, the biological sensor 100 according to this embodiment is provided with the first antenna conductor 11 and the second antenna conductor 12 that is connected to the earth ground 6 via the plant 2. The AC power to be generated between these antenna conductors in accordance with the resistance component 10 of the plant 2 relative to the earth ground 6 is detected. This enables relatively high AC power to be obtained despite a simple configuration, and the state of the plant 2 can be detected easily and with high accuracy.

In this context, for example, a method of observing a state of a plant, the method being different from the method of the present application, is described. In general, in order to observe biopotential of a plant, two electrodes are attached to parts such as a leaf and a stem, and weak electrical signals to be generated between the electrodes are amplified and checked. In this case, energy to be generated between the two electrodes is observed, and hence at least a local variation in potential along, for example, with photosynthesis can be read. Meanwhile, it is unclear what a state of an entirety of the plant is, and it is difficult to translate results of the measurement, for example, into water content of the entirety of the plant.

Further, a method of observing potential of the entirety of the plant relative to the earth ground is also conceived as another method of observing the biopotential of the plant. In this case, of the two electrodes, one is connected to the plant, and another one is connected to the earth. There may be influence of a distance between the plant to be measured and the earth or of weather. In addition, an electrical signal to be measured is weak, and hence is difficult to accurately measure.

Still further, there is a known method of promoting growth or deterring pests by directly applying electrical signals to plants, and a known method of causing the plant to function as a human motion sensor. In these methods, two electrodes are attached, for example, to a plant to be an object, and a DC or an AC signal is applied between the electrodes. Thus, it is difficult to measure, for example, the biopotential together.

In this embodiment, the AC power to be generated between the first antenna conductor 11 and the second antenna conductor 12 that is connected to the earth ground 6 via the plant 2 is detected. In addition, the applied signal is applied from the signal generator 25 to the first antenna conductor 11. Such a configuration functions similar to the monopole antenna in which the plant 2 (resistance component 10 with the resistance value Rt) is interposed between the feed point 7 and the earth ground 6.

Thus, in the biological sensor 100, by detecting the AC power to be generated (at the feed point 7) between the first antenna conductor 11 and the second antenna conductor 12, the variation in the resistance component 10 of the plant 2 can be detected. The resistance value Rt of the resistance component 10 of the plant 2 varies with the water content and electrolyte concentration of the entirety of the plant 2. Thus, by observing the variation in the AC power to be generated at the feed point 7, the state of the entirety of the plant 2 can be determined.

Further, the output of the biological sensor 100 (voltage level of the voltmeter 22) is sufficiently higher, for example, than the biopotential or the like. This enables the variation in the output of the biological sensor 100 to be easily detected. As a result, the variations in the water content and the electrolyte concentration of the plant 2 can be detected with high accuracy. Still further, a dedicated probe electrode, an amplifier circuit, and the like for capturing weak signals such as the biopotential are unnecessary. Thus, the apparatus configuration can be simplified.

The biological sensor 100 induces potential corresponding to the applied signal in the resistance component 10 of the plant 2, and does not directly apply an electrical signal. This presumably enables both visualization of the variation in the resistance component 10 due to the variations in the water content and the electrolyte concentration of the plant 2 itself, and growth promotion or pest deterrence by inducing potential in the plant 2 itself. This enables advantages such as the growth promotion to be achieved while monitoring the state of the plant 2.

### <Second Embodiment>

A biological sensor according to the second embodiment of the present technology is described. Hereinbelow, description of configurations and functions similar to those of the biological sensor 100 described in the above-described embodiment is omitted or simplified.

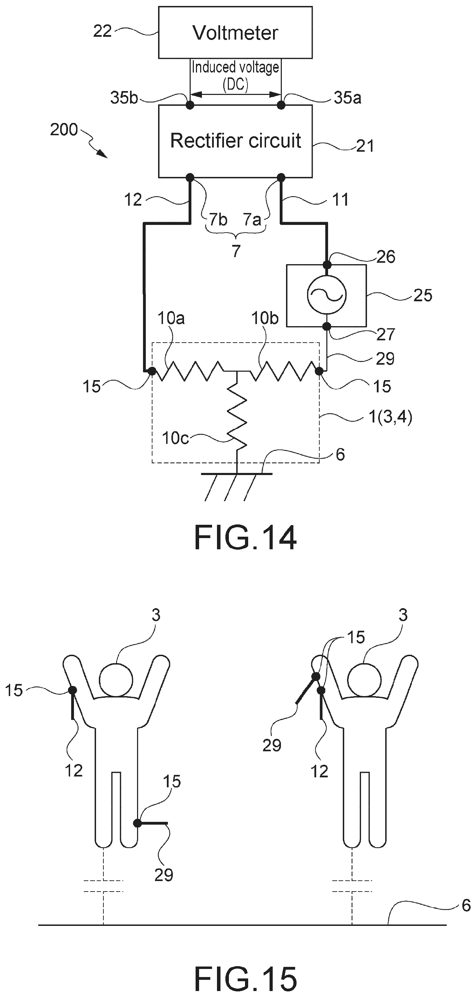

Fig. 14 is a schematic circuit diagram showing an example of a configuration of the biological sensor according to the second embodiment. In this embodiment, the organism 1 to be detected using a biological sensor 200 is a human or an animal. In this case, the second antenna conductor 12 is attached to the body of the human or the body of the animal.

The resistance value Rt of the resistance component 10 (specifically, resistance components 10a, 10b, and 10c) constituting the organism 1 such as the human, the animal, or the like varies in accordance with content of water and concentration of electrolytes (such as blood and lymph fluid) contained in the organism 1. Thus, detection of the variation in the resistance component 10 (10a, 10b, and 10c), specifically, a variation in AC power to be generated in the biological sensor 200 in accordance with the resistance component 10 (10a, 10b, and 10c) enables monitoring of states of the content of the water and the concentration of the electrolytes contained in the human or the animal.

As shown in Fig. 14, a circuit configuration of the biological sensor 200 is substantially similar to, for example, that of the biological sensor 100 for plants, the biological sensor 100 being described with reference to Fig. 8. Not only the second antenna conductor 12, but also the ground terminal 27 of the signal generator 25 is connected to the organism 1 to be an object. Note that, in the biological sensor 200, the attachment portions 15 of the second antenna conductor 12 and the ground terminal 27 are configured to be attachable to the body of the human or the animal. In addition, the organism 1 is connected to the earth ground 6, for example, by capacitive coupling.

The frequency of the applied signal is set in accordance with frequency characteristics of the resistance component 10 (10a, 10b, and 10c) of the organism 1. For example, at a frequency of 1 GHz or less, the resistance component 10 (10a, 10b, and 10c) is low, which enables the body of the human to function as part of an antenna. Thus, when a detection object is the human, an applied signal with 1 GHz or less is used. Further, the amplitude of the applied signal is set as appropriate in accordance, for example, with the resistance value Rt of the resistance component 10 (10a, 10b, and 10c), for example, within a range that does not affect the organism 1. Still further, the frequency or the amplitude of the applied signal may be set in accordance with the characteristics of the organism 1 or application of the biological sensor 200.

In the organism 1 such as the human or the animal, the resistance component 10a that is connected to the second antenna conductor 12, the resistance component 10b that is connected to the ground terminal 27 of the signal generator 25, and the resistance component 10c that connects the resistance component 10a and the resistance component 10b to the earth ground 6 are configured. This is similar to the case of the plant 2 shown in Fig. 8. This enables the signal generator 25 to output the applied signal relative to potential common to the organism 1. Thus, the power level of the detection signal increases. As a result, high detection accuracy can be maintained without interfering with movements or actions of the human or the animal.

Fig. 15 is a schematic view illustrating examples of applying the biological sensor 200 shown in Fig. 14 to the human body. Here, cables that connect the ground terminal 27 and human bodies 3 are referred to as ground cables 29. Fig. 15 schematically illustrates the second antenna conductors 12 and the ground cables 29 that are connected to the human bodies 3 via the attachment portions 15. Note that, the earth ground 6 and the human bodies 3 are capacitively coupled to each other through space, and hence resistance between the earth ground 6 and the human bodies 3 can be regarded as constant.

A left side of Fig. 15 illustrates an example in which the biological sensor 200 shown in Fig. 14 is attached via a wrist and an ankle of the human body 3. Here, although the second antenna conductor 12 is attached to the wrist, and the ground cable 29 is attached to the ankle, the attachment may be reversed. With this, voltage in accordance with water content and electrolyte concentration between the wrist and the ankle is induced at the feed point 7 of the biological sensor 200 shown in Fig. 14. This enables, for example, determination of timings when a wearer drinks or eats, thereby enabling management of a physical condition of the wearer.

Meanwhile, a right side of Fig. 15 illustrates an example of attaching the biological sensor 200 via both ends of muscles in a hand of the wearer. In this example, although the second antenna conductor 12 and the ground cable 29 are attached sequentially from a side close to their torso, the attachment may be reversed. With this, for example, an amount of a variation in the resistance component 10 due to contraction of the muscles can be detected. This enables the biological sensor 200 to be used, for example, like an electromyography sensor. In addition, the second antenna conductor 12 and the ground cable 29 are attached close to each other, and hence, for example, local water content between the respective attachment portions 15 can be detected. In this case, the biological sensor 200 can be used, for example, like a skin sensor.

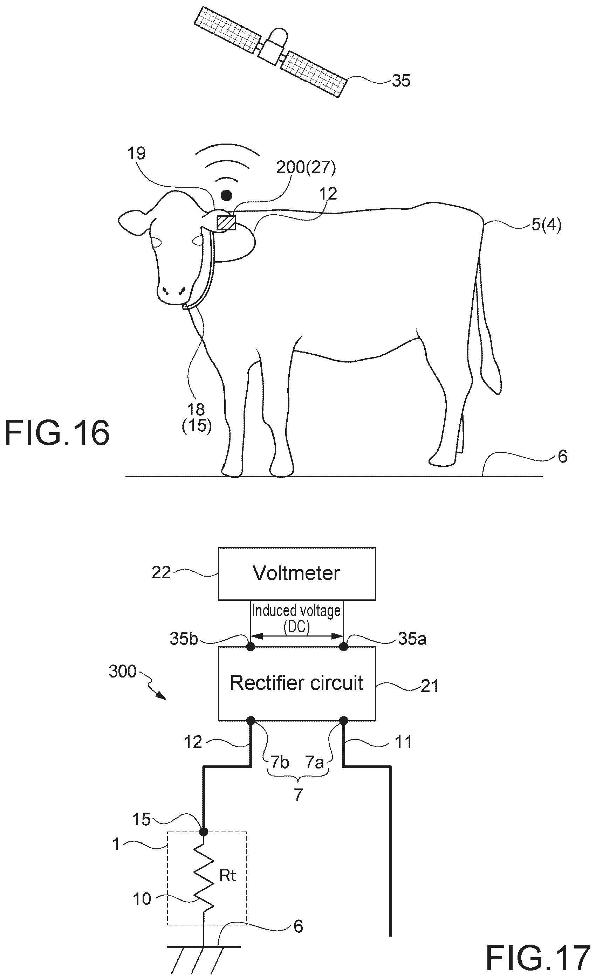

Fig. 16 is a schematic view illustrating an example of applying the biological sensor 200 shown in Fig. 14 to an animal. Here, the biological sensor 200 is attached to an animal 4. This enables monitoring of a state of the animal 4 as in the case of the humans. Here, although a cattle 5 is illustrated as an example of the animal 4, types of the animal 4 are not limited thereto. Application, for example, to livestock such as horses, pigs, and birds, and to wild animals to be observed is also possible. Note that, also in the case of the animal 4, due to the capacitive coupling with the earth ground 6, resistance between the earth ground 6 and the animal 4 can be regarded as constant.

The second antenna conductor 12 of the biological sensor 200 is connected to a collar 18 of the cattle 5. In this case, the collar 18 functions as the attachment portion 15 of the second antenna conductor 12. Meanwhile, the ground terminal 27 of the signal generator 25 is connected, for example, to an ear 19 of the cattle 5 via, for example, the probe electrode with the clip structure. Note that, connection positions of the second antenna conductor 12 and the ground terminal 27 may be reversed. This enables the biological sensor 200 to detect a variation in the resistance component 10 from the neck part to the ear 19 of the cattle 5.

Further, detected data detected by the biological sensor 200 is transmitted to a satellite 35 via a communication module that performs satellite communication. For example, a GPS sensor that acquires a current position of the cattle 5, a temperature sensor that measures an ambient temperature, and the like may be provided together with the biological sensor 200. In this case, together with the detected data of the biological sensor 200 (data indicating the state of the cattle 5), information about the position of the cattle 5, information about the ambient temperature around the cattle 5, and the like can also be transmitted to the satellite 35. This enables, for example, close monitoring and management of the state of the grazed cattle 5 or the like.

Still further, as a power supply of the biological sensor 200, a battery may be used, or vibration power generation utilizing vibrations to be generated by motion of the ear of the cattle 5 may be used. For example, a vibration power generation unit may be provided in a body of the biological sensor 200, and the body with this unit is attached to the ear of the cattle 5. Alternatively, only the vibration power generation unit may be attached to the ear of the cattle 5. This eliminates a need for battery replacement, and enables, for example, provision of a biological management system that is easy to maintain.

### <Other Embodiments>

The present technology is not limited to the embodiments described hereinabove, and may be implemented in various other embodiments.

In the embodiments described hereinabove, the example in which the applied signal is applied from the signal generator 25 to the first antenna conductor 11 is mainly described. This enables utilization of stable applied signals, and hence the state of the organism 1 can be stably detected. Meanwhile, since this configuration is provided with the signal generator 25, a circuit with this configuration may be complicated, or power consumption may increase.

By the way, as described with reference, for example, to Fig. 2, configuring the first antenna conductor 11 as the antenna that receives electromagnetic waves also enables the induction of the AC power in accordance with the resistance component 10 of the organism 1. Thus, configuring the first antenna conductor 11 as appropriate enables biological sensors to be configured without provision of the signal generator 25.

Fig. 17 is a schematic circuit diagram showing an example of a configuration of a biological sensor according to another embodiment. In this biological sensor 300 shown in Fig. 17, the signal generator 25 is not provided, and the first antenna conductor 11 is configured as an antenna that receives electromagnetic waves. Note that, in the biological sensor 300, configurations other than that of the first antenna conductor 11 are similar, for example, to the configurations in the above-described other biological sensors.

The first antenna conductor 11 is set to have a length corresponding to 1/4 of a wavelength of a predetermined AC component among AC components to be induced in the organism 1. Note that, the AC component to be induced in the organism 1 refers, for example, to an AC electrical signal that can be induced in the organism 1, for example, when the organism 1 functions as an antenna. For example, when the organism 1 is a human body, as described above, the human body functions as part of an antenna at the frequency of 1 GHz or less. In other words, at the frequency of 1 GHz or less, the resistance component 10 of the human body is relatively low, and functions as a path that connects the feed point 7 to the earth ground 6. The same applies to animals other than the human body.

When the biological sensor 300 is applied to a human body or an animal, the length of the first antenna conductor 11 is set to have the length corresponding to 1/4 of a wavelength of a preset predetermined AC component among AC components at 1 GHz or less. This enables maximization of efficiency in receiving the predetermined AC component. The predetermined AC component is set in accordance, for example, with frequency characteristics of the resistance component of the organism 1, strength of electromagnetic waves present in a surrounding environment of the organism 1, and the like. For example, by selecting a frequency at which the resistance component of the organism 1 is low, or by selecting a frequency at which the strength of the electromagnetic waves is high, the AC power to be induced in the organism 1 can be increased. Note that, in a frequency band of 1 GHz or more, the resistance value Rt of the resistance component 10 is excessively high. Thus, the organism 1 does not function as part of an antenna.

For example, when a frequency of the predetermined AC component is set to 500 MHz, its wavelength λ is 60 cm, and hence the length of the first antenna conductor 11 is 15 cm. This is an antenna length of the first antenna conductor 11, which is, for example, a length of a current path from the antenna-side feed point 7a to a tip of an antenna. In order to reduce a substantial size of the first antenna conductor 11, there are methods such as winding the antenna with the length of 15 cm in a coil form. The first antenna conductor 11 may be constituted by other methods. For example, the first antenna conductor 11 may be constituted by using a meander line, a chip inductor, or the like.

Note that, the strength of the electromagnetic waves that the biological sensor 300 (first antenna conductor 11) receives may not be constant. Thus, for example, a method of calibrating a reference value for power detection in accordance with a level of the surrounding electromagnetic waves before using the biological sensor 300 is conceived. Alternatively, for example, a method of calibrating output of the biological sensor 300 with reference to the power at the feed point of another monopole antenna that is separately prepared and connected to the earth ground 6 without intermediation of the organism 1 may be employed.

Although the biological sensor 300 shown in Fig. 17 is typically intended for humans and animals that are able to receive electromagnetic waves at a high frequency of approximately 1 GHz, the biological sensor 300 is applicable also to the plant 2. For example, in a case where there is no problem even when a size of a casing is large and the antenna length is large, or in a case where low reception sensitivity is acceptable, the biological sensor 300 with the first antenna conductor 11 that receives the electromagnetic waves may be applied to the plant 2.

In the embodiments described hereinabove, the method of detecting, via the voltmeter 22, the output of the rectifier circuit 21 connected to the feed point 7 is described as a method of detecting the AC power to be generated at the feed point 7 between the first antenna conductor 11 and the second antenna conductor 12. The method of detecting the AC power to be generated at the feed point 7 is not limited thereto.

For example, an ammeter that detects output current of the rectifier circuit 21 may be provided. In other words, a value of DC current to be output from the rectifier circuit 21 is detected as the AC power to be generated at the feed point 7. In this case, the ammeter functions as the DC detection circuit that detects the DC power to be output from the rectifier circuit 21. In such a way, also when the value of the DC current is detected, it is possible to detect the state of the organism 1, the state varying in accordance with the resistance component 10.

In addition, the rectifier circuit 21 need not necessarily be provided, and the AC power to be generated at the feed point 7 may be directly detected. In this case, an AC voltmeter capable of detecting the AC voltage, or an AC ammeter capable of detecting AC current is connected to the feed point 7. Other arbitrary configurations capable of detecting the AC power to be generated at the feed point 7 may be employed.

It is also possible to combine at least two features of the features according to the present technology described hereinabove. In other words, the various features described respectively in the embodiments may be arbitrarily combined with each other irrespective of which embodiment. In addition, the various advantages described hereinabove are merely examples and not limited thereto, and other advantages may be achieved.

The terms same, equal, orthogonal, and the like used herein encompass concepts of substantially the same, substantially equal, substantially orthogonal, and the like. For example, the terms also encompass states that fall within predetermined ranges (such as ranges of ±10%) based on criteria such as completely the same, completely equal, completely orthogonal, and the like.

Note that, the present technology may also employ configurations as follows.
(1) A biological sensor, including:
   a first antenna conductor;
   a second antenna conductor that is a conductor different from the first antenna conductor, and is electrically coupled to an organism connected to an earth ground directly or by capacitive coupling; and
   a power detection unit that detects AC power to be generated between the first antenna conductor and the second antenna conductor in accordance with a resistance component of the organism relative to the earth ground.
(2) The biological sensor according to (1), further including
   a signal generator that applies a predetermined AC signal to the first antenna conductor.
(3) The biological sensor according to (2), in which
   a frequency of the predetermined AC signal is set in accordance with frequency characteristics of the resistance component of the organism relative to the earth ground.
(4) The biological sensor according to (3), in which the organism is a plant, and
   the frequency of the predetermined AC signal is set to a frequency of 5 Hz or more and 3 MHz or less.
(5) The biological sensor according to any one of (2) to (3), in which
   the predetermined AC signal has any of a rectangular wave, a sine wave, and a triangular wave.
(6) The biological sensor according to any one of (2) to (5), in which
   the signal generator includes a ground portion that provides reference potential for the predetermined AC signal, and
   the ground portion is electrically coupled to the organism.
(7) The biological sensor according to any one of (2) to (5), in which
   the signal generator includes a ground portion that provides reference potential for the predetermined AC signal, and
   the ground portion is connected to the earth ground.
(8) The biological sensor according to any one of (2) to (5), in which
   the signal generator includes a ground portion that provides reference potential for the predetermined AC signal, and
   the ground portion is in a state electrically floating from the earth ground.
(9) The biological sensor according to any one of (1) to (8), in which
   the power detection unit includes
   a rectifier circuit that rectifies the AC power to be generated between the first antenna conductor and the second antenna conductor, and
   a DC detection circuit that detects DC power to be output from the rectifier circuit.
(10) The biological sensor according to (9), in which
   the DC detection circuit is either one of a voltmeter that detects output voltage of the rectifier circuit, and an ammeter that detects output current of the rectifier circuit.
(11) The biological sensor according to (9), in which
   the DC detection circuit is a voltmeter that detects output voltage of the rectifier circuit, and
   an internal resistor of the voltmeter is adjusted to achieve predetermined sensor sensitivity.
(12) The biological sensor according to (11), in which
   the organism is a plant, and
   a resistance value of the internal resistor of the voltmeter is 1 kQ or more and 10 MΩ or less.
(13) The biological sensor according to any one of (1) to (12), in which
   the organism is a plant, and
   the second antenna conductor is attached to an aboveground part of the plant.
(14) The biological sensor according to (13), in which
   the second antenna conductor has either one of a clip structure that presses an end portion of the second antenna conductor against an outside of the plant, and a needle structure that inserts the end portion of the second antenna conductor into an inside of the plant.
(15) The biological sensor according to any one of (1) to (3) and (5) to (11), in which
   the organism is a human or an animal, and
   the second antenna conductor is attached to the body of the human or the body of the animal.
(16) The biological sensor according to any one of (1) to (15), in which
   the first antenna conductor is set to have a length corresponding to 1/4 of a wavelength of a predetermined AC component among AC components to be induced in the organism.
(17) The biological sensor according to any one of (1) to (16), in which
   an energy harvesting apparatus is used as a power supply.
(18) A biological management system, including:
   at least one biological sensor including
      a first antenna conductor,
      a second antenna conductor that is a conductor different from the first antenna conductor, and is electrically coupled to an organism connected to an earth ground directly or by capacitive coupling, and
      a power detection unit that detects AC power to be generated between the first antenna conductor and the second antenna conductor in accordance with a resistance component of the organism relative to the earth ground; and
   a management apparatus that performs a management operation on the basis of a result of detection by the at least one biological sensor.

### Reference Signs List

- 1: organism
- 2: plant
- 3: human body
- 4: animal
- 6: earth ground
- 7: feed point
- 10: resistance component
- 11: first antenna conductor
- 12: second antenna conductor
- 20: power detection unit
- 21: rectifier circuit
- 22: voltmeter
- 25: signal generator
- 27: ground terminal
- 28: power supply
- 45: internal resistor
- 51: management apparatus
- 100, 200, 300: biological sensor
- 110: biological management system

## Claims

1. A biological sensor, comprising:
a first antenna conductor;
a second antenna conductor that is a conductor different from the first antenna conductor, and is electrically coupled to an organism connected to an earth ground directly or by capacitive coupling; and
a power detection unit that detects AC power to be generated between the first antenna conductor and the second antenna conductor in accordance with a resistance component of the organism relative to the earth ground.

2. The biological sensor according to Claim 1, further comprising
a signal generator that applies a predetermined AC signal to the first antenna conductor.

3. The biological sensor according to Claim 2, wherein
a frequency of the predetermined AC signal is set in accordance with frequency characteristics of the resistance component of the organism relative to the earth ground.

4. The biological sensor according to Claim 3, wherein
the organism is a plant, and
the frequency of the predetermined AC signal is set to a frequency of 5 Hz or more and 3 MHz or less.

5. The biological sensor according to Claim 2, wherein
the predetermined AC signal has any of a rectangular wave, a sine wave, and a triangular wave.

6. The biological sensor according to Claim 2, wherein
the signal generator includes a ground portion that provides reference potential for the predetermined AC signal, and
the ground portion is electrically coupled to the organism.

7. The biological sensor according to Claim 2, wherein
the signal generator includes a ground portion that provides reference potential for the predetermined AC signal, and
the ground portion is connected to the earth ground.

8. The biological sensor according to Claim 2, wherein
the signal generator includes a ground portion that provides reference potential for the predetermined AC signal, and
the ground portion is in a state electrically floating from the earth ground.

9. The biological sensor according to Claim 1, wherein
the power detection unit includes
a rectifier circuit that rectifies the AC power to be generated between the first antenna conductor and the second antenna conductor, and
a DC detection circuit that detects DC power to be output from the rectifier circuit.

10. The biological sensor according to Claim 9, wherein
the DC detection circuit is either one of a voltmeter that detects output voltage of the rectifier circuit, and an ammeter that detects output current of the rectifier circuit.

11. The biological sensor according to Claim 9, wherein
the DC detection circuit is a voltmeter that detects output voltage of the rectifier circuit, and
an internal resistor of the voltmeter is adjusted to achieve predetermined sensor sensitivity.

12. The biological sensor according to Claim 11, wherein
the organism is a plant, and
a resistance value of the internal resistor of the voltmeter is 1 kQ or more and 10 MΩ or less.

13. The biological sensor according to Claim 1, wherein
the organism is a plant, and
the second antenna conductor is attached to an aboveground part of the plant.

14. The biological sensor according to Claim 13, wherein
the second antenna conductor has either one of a contact structure that presses an end portion of the second antenna conductor against an outside of the plant, and a needle structure that inserts the end portion of the second antenna conductor into an inside of the plant.

15. The biological sensor according to Claim 1, wherein
the organism is a human or an animal, and
the second antenna conductor is attached to a body of the human or a body of the animal.

16. The biological sensor according to Claim 1, wherein
the first antenna conductor is set to have a length corresponding to 1/4 of a wavelength of a predetermined AC component among AC components to be induced in the organism.

17. The biological sensor according to Claim 1, wherein
an energy harvesting apparatus is used as a power supply.

18. A biological management system, comprising:
at least one biological sensor including
a first antenna conductor,
a second antenna conductor that is a conductor different from the first antenna conductor, and is electrically coupled to an organism connected to an earth ground directly or by capacitive coupling, and
a power detection unit that detects AC power to be generated between the first antenna conductor and the second antenna conductor in accordance with a resistance component of the organism relative to the earth ground; and
a management apparatus that performs a management operation on a basis of a result of detection by the at least one biological sensor.
